# EUROPEAN PATENT APPLICATION

(11) **EP 1 457 189 A1**
(43) Date of publication of application: **15.09.2004**
(21) Application number: 03005249.2
(22) Date of filing: 10.03.2003
(51) Int. Cl.: A61H 39/00, A61N 1/36

(54) **An electric massaging pad having dual electrodes**

(71) Applicant: Chen, Yi-Ying, Vancouver, British Columbia (CA)
(72) Inventor: Wang, Wei-Cheng, Panchiao City, Taipei Hsien (TW)
(74) Representative: Casalonga, Axel

(57) **Abstract**

An electronic massaging apparatus for application over selected spots of the body is described. The apparatus has an attaching pad with dual electrodes (11,12) properly isolated from the each other. When the apparatus is activated, an electronic pulse train carrying a small current at low voltage enters the subcutaneous layer of the human body through the electrodes (11,12). When the current passes through the body tissues with impedance matching, the electrical stimuli cause rhythmic contraction of the muscles. The invention using the dual electrode pad allows the apparatus to be focused on localized spots or acupuncture points for electrical stimulation. Also, the area of body tissues to reach impedance matching can be reduced, therefore it only takes a small current to drive through the body tissues, thus improving the massaging effect and the safety application of the apparatus.

## Description

### 1. Field of the Invention

The present invention relates to an electronic massaging apparatus having dual electrodes attaching on a single pad that can be applied massage or acupuncture therapy over a selected spot of the body. The selected spot is said to be at the acupuncture point or at the pressure point. The dual electrodes pad, with the connection to a stimulator, e.g. TENSE or nervo-muscular stimulator, that will produce the massaging effect.

### 2. Description of Related Arts

In contemporary society, people commonly suffer from symptoms such as stomach ache, insomnia, and muscle strains due to pressure at the work place or the living environment in general. For example, lumbago or pain in the lumbar region is largely caused by straining of the muscles while sitting down for a prolonged period of time without changing the posture. One way of correcting the muscle strain is through some kind of physical treatment such as a massaging chair, or body massage service. However, these pressure-relieving methods are sometimes not very practical, because the body massage could be time consuming and can create a burden for the personal budget, and a massaging chair could occupy a lot of space in a small house.

At present, a conventional type of electronic nervo-muscular stimulation device is available that is applied over selected spots of the human body to cause rhythmic contraction of the muscles under the electrical stimuli with a small current at low voltage. In Fig. 9, the architecture of the device includes a main unit (70), and a pair of electrode plates (71, 72), and the output of the main unit (70) is connected to the electrodes (71, 72) through a lead wire (73). Currently available nervo-muscular stimulation devices all have two separate attaching pads each carrying an electrode plate (71, 72). The electrode plates (71) (72) are respectively connected to one branch of the lead wire (73), whilst the other end of the lead wire is connected to the main unit (70).

The two electrode plates (71, 72) are made from conductive rubber materials that can be attached over selected spots of the skin for massaging. Since the two electrode plates (71, 72) are physically separated from each other, when one of them is applied over the skin, there is no current, and only when two electrode plates (71, 72) are simultaneously applied on the skin, for example attaching over the shoulder as shown in Fig. 9, an electronic pulse train is then emitted from the main unit (70) and enters the subcutaneous layer between the two electrodes with impedance matching. The electrical stimuli cause the muscles in the region to contract rhythmically in accordance with the pulse frequency thus producing the massaging effect.

However, the above mentioned electronic stimulation device has certain shortcomings to be described below:
(1) Difficult to focus on a spot: Since the two electrode plates have to be placed over the human body at the same time and on the same area to activate the electronic device, it will be difficult to position it precisely over the acupuncture points of the body for massaging.
(2) Excessive power consumption: Since the two electrode plates are physically separated from each other, when applied over the body, it requires a large current at a relatively high voltage to drive the current through the body tissues.

The main object of the present invention is to provide an electronic massaging apparatus for massaging at a selected spot of the body. The apparatus using a dual electrodes pad allows it to be precisely placed over a selected spot of the body for stimulating acupuncture points.

The construct of electronic massaging apparatus in accordance with the present invention comprises a first electrode and a second electrode constructed on a dielectric layer, such that the first and second electrode are properly isolated from each other during operation.

When the electronic pulse train is emitted from the control unit, the pulse train enters the subcutaneous layer of the body through one of the electrodes and returns to the control unit through the other electrode forming a closed loop, and the localized electrical stimulation on a selected spot allows the apparatus to be focused on a small spot for stimulating the acupuncture points underneath the skin.

The above mentioned first electrode is round in shape, and the second electrode is ring shaped coaxially surrounding the first electrode, wherein the inner diameter of the second electrode is larger than the outer diameter of the first electrode. When the apparatus is activated, an electronic pulse train passes through the gap between the two electrodes to form a closed loop

The present invention is further characterized in that the first and second electrode plates could take on any circular shape coaxially or could be divided up into two hemispheres but both electrodes should be formed on a dielectric layer shared in common by the electrodes.

The present invention is further characterized in that the first and second electrode plates are formed by conductive rubber coating on the dielectric layer.

The present invention is further characterized in that the first and second electrode plates are each connected to an electrically conductive ring that allows a retaining stem to pass through the hollow space in the center and penetrate the dielectric layer to the outside for making the necessary electrical connection with the lead wire leading to the control unit.

### IN THE DRAWINGS:

Figs. 1A and 1B are the top and bottom views of the first embodiment of the present invention;
Figs. 2A-C are the plan views of different configurations that can be derived from the present invention;
Fig. 3 is a plan view of another configuration for the present invention;
Figs. 4-8 reveal the important acupuncture points over the human body; and
Fig. 9 is an application diagram of a conventional nervo-muscular stimulation device.

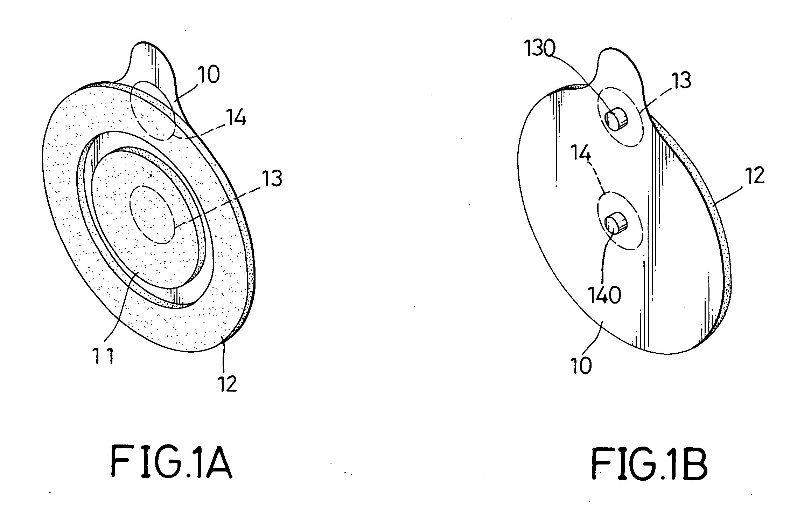

The present invention provides an electronic massaging apparatus having a dual electrodes on a single pad, as shown in Figs 1A and 1B, for applying over a selected spot of the body to produce the massaging effect, where the two electrodes are properly isolated from each other during operation. The main focus of the present invention is that the dual electrode pad allows the electronic massaging apparatus to be precisely placed the pad over a selected spot of the body to act on acupuncture points underneath the skin for optimized massaging effect.

The construct of the dual electrode pad comprises a first electrode (11), a second electrode (12) both created on a dielectric layer (10) shared in common by the electrodes (11, 12), and the first and second electrodes (11, 12) are properly isolated from each other in operation.

When the electronic pulse train is outputted from the control unit (not shown), the pulses enters the subcutaneous layer of the body through one of the electrodes and returns to the control unit through the other electrode forming a closed loop. The electrical stimuli cause rhythmic contraction of the muscles in the selected region to produce the massaging effect. With the special dual electrode construct, the apparatus can be precisely placed over selected spots over the body to stimulate acupuncture points underneath the skin for optimized massaging effect.

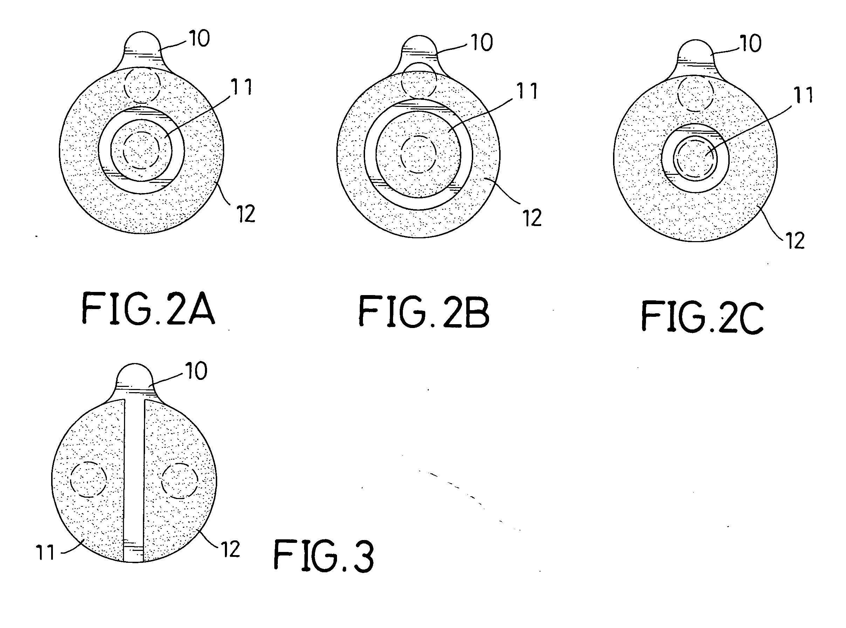

The first electrode (11) is round in shape, and the second electrode (12) is ring shaped coaxially surrounding the first electrode, as shown in Figs. 2A∼C, but both are properly isolated from each other during operation, wherein the inner diameter of the second electrode (12) is greater than the outer diameter of the first electrode (11), leaving a small gap between the two electrodes (11, 12). When the apparatus is activated, the electronic pulse can easily jump over the gap between the two electrodes (11, 12) and form a closed loop carrying a small current at low voltage. The area of body tissue subjecting to electrical stimuli with impedance matching varies according to the diameter of the electrodes (11, 12) used and the gap between the electrodes (11, 12). Other possible variations are shown in Figs. 2A, 2B and 2C.

In Fig. 2A, the outer diameter of the first electrode (11) is closest to the inner diameter of the second electrode (12) among all three cases, so the gap between electrodes (11, 12) is smallest. When a current is supplied through the electrodes (11, 12), it enters the subcutaneous layer with impedance matching to form a closed loop carrying a small current, and the electrical stimuli act on the acupuncture points underneath the skin for body massaging.

In Fig. 2B, the outer diameter of the first electrode (11) and the inner diameter of the second electrode (12) are somewhat smaller than those seen in Fig. 2A, the stimulation area is therefore smaller than that in the first case. In Fig. 2C, the outer diameter of the first electrode (11) and the inner diameter of the second electrode (12) are even smaller than those seen in Fig. 2B, thus, the stimulation area is smallest among all but most intensely focused on a localized spot.

In Figs. 2A∼2C, in all three cases illustrated, the round shaped electrode (11) and the ring shaped electrode (12) are constructed coaxially, but they can take on any geometric shapes for different requirements. For example, the first and second electrodes (11, 12) can be arranged in tandem as shown in Fig. 3. Besides, the intensity of the electronic pulses can be set to a fixed level or with a variable dial, and the stimulation current can be DC/AC, all depending on the needs for different applications.

The electrical connection between the electrodes and the control unit can be implemented in the same way as shown in Figs. 1A and 1B, where two conductive rings (13, 14) are formed on the dielectric layer (10), such that each of the conductive rings (13, 14) is respectively connected to the first or the second electrode (11, 12) and each has a retaining stem (130, 140) passing through the hollow space in the center and penetrating the dielectric layer (10) to the outside for making the necessary connection with the button shaped connectors leading to the control unit.

The amount of current passing through the human body can be calculated by multiplying the contact area and the amount of current flowing pass per unit area (mA/cm2), where the current supplied by the apparatus is 1 milliampere (mA). For example, if the diameter of the contact area of the electrode plate is 5 cm, then the amount of current can be calculated using the above formula to be 0.05 mA/cm2.

The amount of current and the voltage level generated through the electrodes can be explained using the analogy of water flowing through and shooting out of the pipes. When the water pressure is kept at the same level, the length of the water column ejected from a larger diameter pipe will be shorter than that through a smaller diameter pipe, but the rate of flow will be proportional to the diameter of the pipe. Thus, the design of the dual electrode on an attaching pad allows the electrical stimuli to be concentrated on a small area with deeper penetration, as compared with other conventional nervo-muscular devices using two attaching pads.

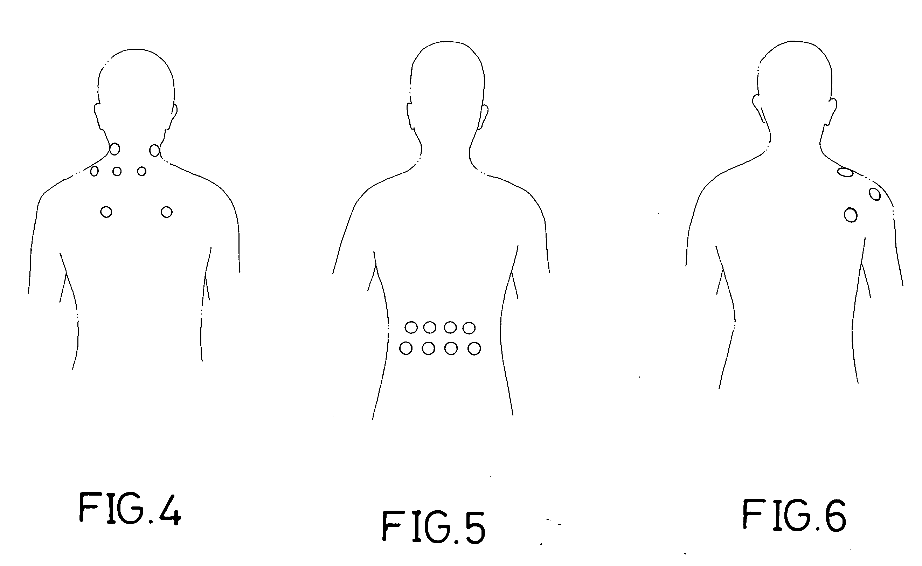

In accordance with the present invention, the dual electrode construct allows the apparatus to be placed over a selected spot of the body for precise stimulation of the acupuncture point, as these acupuncture points are known in Chinese medicine to be capable of governing the functions of various parts of the human body. Some of the important ones are illustrated in Figs. 4~8. The present invention can help realize precise acupuncture without invasive application of the needles.

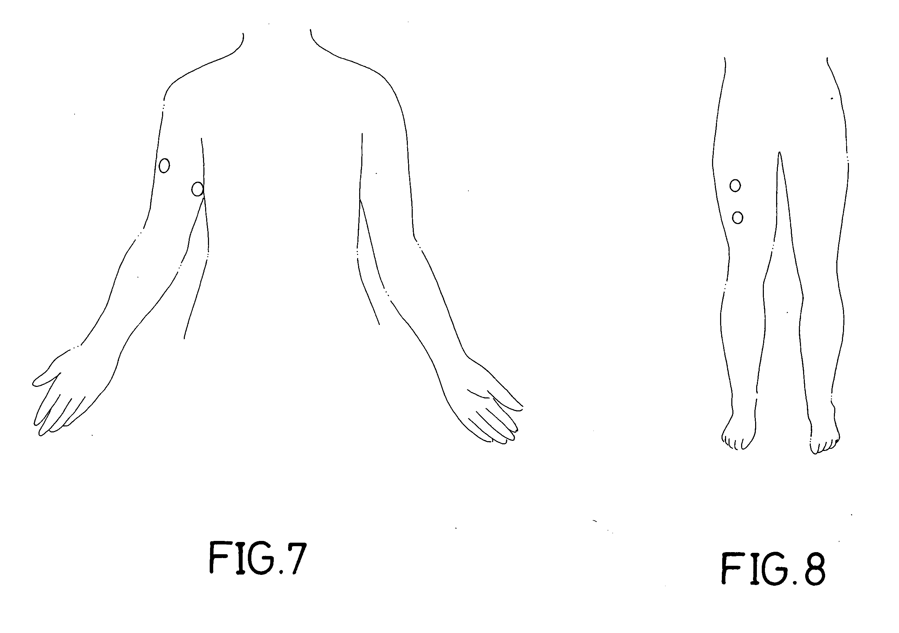

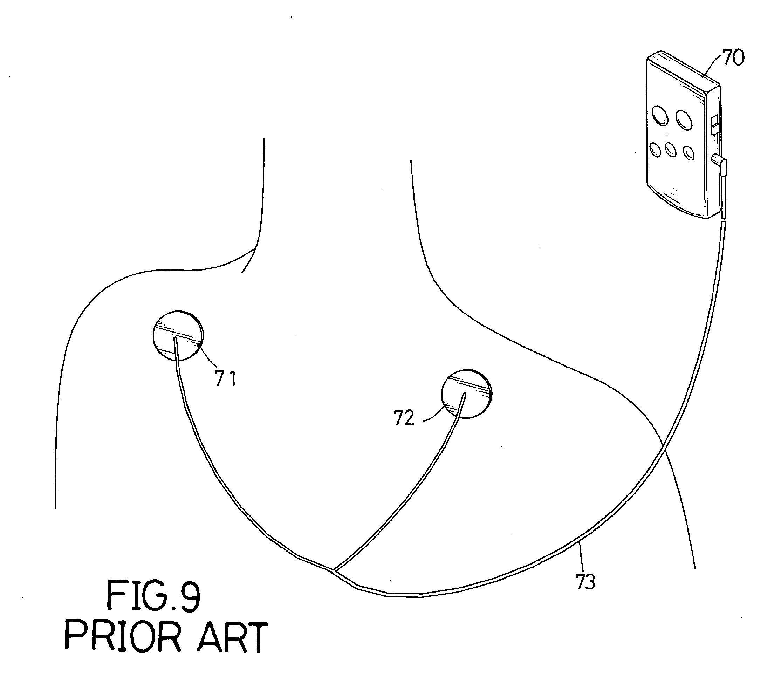

Fig. 4 shows the acupuncture points over the neck and back regions governing the circulation and the function of the gall bladder; Fig. 5, the acupuncture points near the lumbar region governing the functions of the intestine, gall bladder, and liver; Fig. 6, the acupuncture points around the shoulder region governing the functions of the gall bladder and intestine; Fig. 7, the acupuncture points around the forearm region governing the functions of the lung and heart; and Fig. 8, the acupuncture points around the leg region governing the functions of the stomach and spleen.

It will be appreciated that the shape and configuration of the electrodes can be appropriately changed and adjusted by a person with ordinary skill in the art without departing from the scope of the invention. The foregoing description of the preferred embodiments of the present invention is intended to be illustrative only and, under no circumstances, should the scope of the present invention be so restricted.

## Claims

1. An electronic massaging apparatus having dual electrodes on a single pad, comprising a first electrode (11) and a second electrode (12), both created on a dielectric layer (10) shared in common by the first and second electrodes (11,12), and the first and second electrodes (11,12) are properly isolated from each other and connected to the output of control unit through a connector.

2. The electronic massaging apparatus as claimed in claim 1, wherein the first electrode (11) and the second electrode (12) are both circular and one of the electrodes encircles the other electrode.

3. The electronic massaging apparatus as claimed in claim 2, wherein the first electrode (11) is round in shape and the second electrode (12) is ring shaped and surrounding the first electrode (11), and both the electrodes (11,12) are properly isolated from each other in operation.

4. The electronic massaging apparatus as claimed in claim 1, wherein the first and second electrodes (11,12) can take on any circular shape coaxially or can be divided up into two hemispheres, provided that both electrodes are formed over a dielectric layer shared in common.

5. The electronic massaging apparatus as claimed in claim 1, wherein two conductive rings (13,14) are formed on the dielectric layer (10), such that each conductive ring (13,14) is connected to the first or second electrode (11,12), and each has a retaining stem (130,140) passing through a hollow space in'the center of each conductive ring (13,14) and penetrating the dielectric layer (10) to exterior of the dual electrode pad for making necessary connection with lead wire leading to a control unit.

6. The electronic massaging apparatus as claimed in claim 1, wherein the first and second electrodes (11,12) are formed by conductive rubber coating on the dielectric layer (10).

7. The electronic massaging apparatus as claimed in claim 2, wherein the first and second electrodes (11,12) are formed by conductive rubber coating on the dielectric layer (10).

8. The electronic massaging apparatus as claimed in claim 3, wherein the first and second electrodes (11,12) are formed by conductive rubber coating on the dielectric layer (10).

9. The electronic massaging apparatus as claimed in claim 4, wherein the first and second electrodes (11,12) are formed by conductive rubber coating on the dielectric layer (10).

10. The electronic massaging apparatus as claimed in claim 5, wherein the first and second electrodes (11,12) are formed by conductive rubber coating on the dielectric layer (10).
